# FASCICULE DE BREVET EUROPEEN

(11) **EP 0 705 243 B1**
(45) Date de publication et mention de la délivrance du brevet: **24.06.1998**
(21) Numéro de dépôt: 94919723.0
(22) Date de dépôt: 16.06.1994
(51) Int. Cl.: C07C 323/32, C07C 319/20

(54) **BETA-AMINOVINYLCETONES, UN PROCEDE DE PREPARATION ET LEUR UTILISATION DANS LA PREPARATION DE BETA-DICETONES**
BETA-AMINOVINYLKETONE, VERFAHREN ZUR HERSTELLUNG UND IHRE VERWENDUNG BEI DER HERSTELLUNG VON BETA-DIKETONEN
BETA-AMINOVINYL KETONES, PREPARATION METHOD THEREFOR AND USE THEREOF FOR PREPARING BETA DIKETONES

(30) Priorité: 23.06.1993 FR 9307867
(43) Date de publication de la demande: 10.04.1996
(73) Titulaire: RHONE-POULENC AGROCHIMIE, 69009 Lyon (FR)
(72) Inventeur: CASADO, Michel, F-69360 Saint-Symphorien-d'Ozon (FR); LE ROY, Pierre, F-69003 Lyon (FR); PEVERE, Virginie, F-69008 Lyon (FR)
(86) Numéro de dépôt international: FR9400726
(87) Numéro de publication internationale: WO9500476

(56) Documents cités:
- EP-A- 0 487 870
- JOURNAL OF ORGANIC CHEMISTRY OF THE USSR,, vol.26, no.10, Octobre 1990 New York, US, pages 1801 - 1805 V. YA. SOSNOVSKIKH, ET AL.: 'Condensation of nitriles with methyl ketones as a method for the synthesis of beta-aminovinyl ketones'
- JOURNAL OF ORGANIC CHEMISTRY OF THE USSR,, vol.22, no.4, Avril 1986 New York, US, pages 790 - 791 V. YA. SOSNOVSKIKH, ET AL.: 'Condensation of nitriles of aromatic acids with methyl tert-alkyl ketones'
- JOURNAL OF ORGANIC CHEMISTRY,, vol.53, no.11, 27 Mai 1988 Washington, DC, US, pages 2426 - 2429 A. GUARNA, ET AL.: 'Rearrangement of isoxazoline-5-spiro derivatives. 1. Synthesis of 4,5-dihydroisoxazole- 5-spirocyclopropanes and their rearrangement to 5,6-dihydro-4-pyridones'
- JOURNAL OF THE CHEMICAL SOCIETY, PERKIN TRANSACTIONS 1,, no.7, Juillet 1985 Letchworth, GB, pages 1401 - 1406 M. NITTA, ET AL.: 'Metal-carbonyl-induced reaction of isoxazoles. Ring cleavage and reduction by hexacarbonylmolybdenum, pentacarbonyliron, or nonacarbonyldi-iron'
- LA CHIMICA E L'INDUSTRIA,, vol.56, no.1, Janvier 1974 Milan, IT, pages 25 - 26 P. BARVO, ET AL.: 'Preparation and applications of triphenylphosphonium isoxazolylmethylides'

## Description

La présente invention concerne de nouvelles β-aminovinylcétones ayant un substituant aromatique sur le carbone attaché au groupe amino, un procédé de préparation de ces composés à partir de nitriles et d'une méthylorganocétone et leur utilisation pour la préparation de β-dicétones aromatiques.

### Contexte technique de l'invention

Les β-dicétones aromatiques de formule générale: 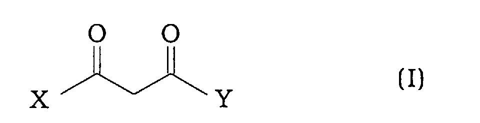 dans laquelle le symbole X représente par exemple un groupe aliphatique ou cycloaliphatique et Y représente par exemple un groupe aromatique, peuvent servir avantageusement de composés intermédiaires dans la synthèse de dérivés de 4-benzoylisoxazoles de formule générale : 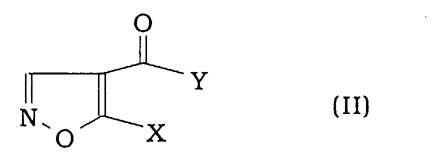 qui peuvent être des herbicides à spectre large, bien connus dans l'état antérieur de la technique (cf. en particulier EP-A 0 418 175).

La synthèse d'un dérivé de 4-benzoyl-isoxazole au départ d'une dicétone de formule (I) peut être conduite en enchaînant les deux étapes suivantes :
- réaction de la dicétone de formule (I) avec un orthoformiate de trialkyle ou un dialkylacétal de diméthylformamide pour conduire à un produit répondant à la formule générale : 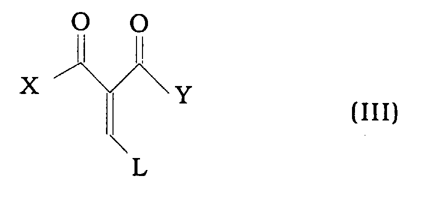 dans laquelle L est un groupe 0-alkyle ou un groupe N,N-dialkylamino ; généralement cette réaction est opérée dans un solvant inerte tel que par exemple l'éther diéthylique ou le tétrahydrofuranne à une température allant de 0°C à la température de reflux du mélange ; et
- réaction du composé de formule (III) avec un sel d'hydroxylamine ; généralement cette réaction est opérée dans un solvant tel que par exemple l'éthanol ou l'acétonitrile, facultativement en présence d'une base ou d'un accepteur d'acide comme par exemple la triéthylamine ou l'acétate de sodium.

En ce qui concerne la β-dicétone de départ de formule (I), sa synthèse est réalisée couramment en enchaînant les trois étapes suivantes :
- réaction d'hydrolyse d'un nitrile aromatique de formule générale :

   Y―CN (IV)

   pour donner l'acide carboxylique correspondant ;
- préparation d'un ester de cet acide, dérivé d'un alcool aliphatique tel que par exemple le méthanol ; et
- réaction de condensation dudit ester sur une cétone de formule générale : 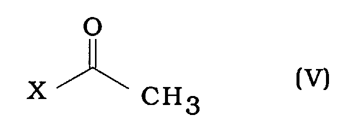 cette réaction étant opérée en présence d'une base forte dans des conditions conformes à la réaction de Claisen. La condensation de nitriles avec des cétones en présence de base forte est décrite notamment dans le Journal of Organic Chemistry of USSR, 26, 1801-5 (1990), mais dans ce cas, on obtient des hydroxynitriles. De plus, ce document précise que le méthoxide de sodium n'est pas utile pour la préparation de dicétones.

### But de la présente invention

La présente invention a pour but essentiel celui de proposer :
- une voie d'accès partant d'un nitrile aromatique et conduisant aux β-dicétones susceptibles de servir de composés intermédiaires dans la synthèse de dérivés de 4-benzoyl-isoxazoles,
- qui présente de nombreux avantages : un nombre restreint d'étapes, des rendements excellents et une réelle facilité de développement à l'échelon industriel.

Selon cette voie d'accès, on prépare une β-aminovinylcétone ayant un substituant aromatique sur le carbone attaché au groupe amino, qui présente la double caractéristique d'être un composé d'une part nouveau et d'autre part pouvant être aisément transformé en β -dicétone souhaitée en soumettant à la réaction de transformation soit l'aminovinylcétone isolée du milieu réactionnel lui ayant donné naissance, soit directement ledit milieu réactionnel renfermant l'aminovinylcétone.

### Description de la présente invention

I - Dans son premier objet, la présente invention concerne une β-aminovinylcétone de formule générale : 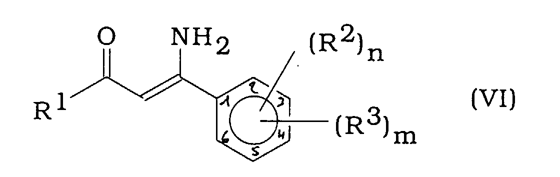 dans laquelle :
- R¹ représente :
   * un groupe alcényle ou alcynyle à chaîne droite ou ramifiée contenant jusqu'à 6 atomes de carbone qui est facultativement substitué avec un ou plusieurs atome(s) d'halogène et dont l'insaturation n'est pas conjugée avec la double liaison cétonique;
   * un groupe cycloalkyle contenant 3 à 6 atomes de carbone, éventuellement substitué avec un ou plusieurs groupe(s) R⁴ ou OR⁴ ou un ou plusieurs atome(s) d'halogène ;
   * un groupe cycloalcényle contenant 4 à 6 atomes de carbone, éventuellement substitué avec un ou plusieurs groupe(s) R⁴ ou OR⁴ ou un ou plusieurs atome(s) d'halogène, dans lequel l'insaturation éthylénique n'est pas conjuguée avec la double liaison cétonique ;
   * un groupe de formule (R⁵ 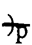 phényl(̵CR⁶R⁷)_{q}-;
   * R⁴ étant un groupe alkyle à chaîne droite ou ramifiée contenant jusqu'à 4 atomes de carbone ;
   * R⁵ étant un groupe alkyle à chaîne droite ou ramifiée contenant jusqu'à 4 atomes de carbone ou un atome d'halogène (F, Cl, Br, I) ;
   * R⁶ et R⁷, qui peuvent être identiques ou différents, représentant chacun un groupe alkyle à chaîne droite ou ramifiée contenant jusqu'à 4 atomes de carbone ;
   * p et q étant des nombres entiers, qui peuvent être identiques ou différents, allant de 0 à 3 ;
- R² représente un substituant attracteur d'électrons choisi dans le groupe formé par :
   * un groupe nitro ;
   * un groupe acyle COR⁴ ;
   * un groupe COOR⁸ ;
   * un groupe alkylthio SR⁴ ;
   * un groupe carbamoyle CONR⁸R⁹ ou thiocarbamoyle CSNR⁸R⁹ ;
   * un groupe halogénoalkyle ou halogénoalkoxy linéaire ou ramifié ayant jusqu'à 4 atomes de carbone et dans lequel la substitution halogéno est une monosubstitution qui peut aller jusqu'à une polysubstitution quasi totale ;
   * R⁴ ayant les définitions générales indiquées ci-avant ;
   * R⁸ et R⁹, qui peuvent être identiques ou différents, représentant chacun un atome d'hydrogène ou un groupe alkyle à chaîne droite ou ramifiée contenant jusqu'à 4 atomes de carbone ;
- R³ représente :
   * un groupe alkyle ou alkoxy à chaîne droite ou ramifiée contenant jusqu'à 4 atomes de carbone, le groupe alkyle pouvant éventuellement être substitué avec un groupe OR⁴ ;
   * un atome d'halogène ;
   * un groupe de formule O(̵CH₂)ᵣ-OR⁴ ;
   * R⁴ possédant la définition générale indiquée ci-avant et r étant un nombre entier allant de 1 à 4;
- le symbole n représente un nombre entier allant de 0 à 2 et le symbole m représente un nombre entier allant de 0 à 3 ;
- avec les réserves a) et b) suivantes :
   a) R⁵ est différent de l'atome de chlore quand R³ est l'atome de chlore et q = 0,
   b) quand n, m et q sont tous égaux à zéro, alors p est différent de zéro.

Comme β-aminovinylcétones préférentiellement représentatives de la présente invention prise dans son premier objet, on peut citer celles qui répondent à la formule (VI) dans laquelle le symbole n représente un nombre allant de 1 à 2 et le symbole m représente un nombre allant de 0 à 3.

Comme β-aminovinylcétones plus préférentiellement représentatives, on peut citer les composés de formule (VI), appartenant au groupe préférentiellement représentatif visé dans le paragraphe qui précède, dans la structure desquels :
- R¹ représente un groupe cycloalkyle contenant 3 à 6 atomes de carbone, éventuellement substitué avec un ou plusieurs groupe(s) R⁴ ou OR⁴ ;
- R² représente un groupe alkylthio SR⁴, un groupe halogénoalkyle ou halogénoalkoxy linéaire ou ramifié ayant jusqu'à 4 atomes de carbone et dans lequel la substitution halogéno est une monosubstitution qui peut aller jusqu'à une polysubstitution quasi totale ;
- R³ représente un groupe alkyle ou alkoxy à chaîne droite ou ramifiée contenant jusqu'à 4 atomes de carbone ou un atome d'halogène.

Comme β-aminovinylcétones encore plus préférentiellement représentatives, on peut citer les composés, appartenant au groupe plus préférentiellement représentatif visé dans le paragraphe qui précède, dans la structure desquels :
- le symbole n représente un nombre entier égale à 1 ou 2 et le symbole m représente un nombre entier allant de 0 à 2, avec la condition supplémentaire selon laquelle la somme m + n est au plus égale à 3 ;
- le (ou l'un des) groupe(s) représenté(s) par (R²)ₙ est en position 2 sur le noyau phényle tandis que le (ou les) éventuelle(s) autre(s) groupe(s) représenté(s) par (R²)ₙ et/ou (R³)ₘ sont en position 3 et/ou 4 sur le noyau phényle.

Comme β-aminovinylcétones tout particulièrement représentatives, on peut citer les composés de formule (VI), appartenant au groupe encore plus préférentiellement représentatif visé dans le paragraphe qui précède, dans la structure desquels :
- R¹ représente un groupe cyclopropyle ou 1-méthylcyclopropyle ;
- R² représente un groupe méthylthio ou éthylthio ou un groupe trifluorométhyle ou trifluorométhoxy ;
- R³ représente un groupe méthyle, éthyle, méthoxy ou éthoxy ou un atome de chlore, de brome ou de fluor.

Comme exemples spécifiques de β-aminovinylcétones, on citera notamment les composés suivants :
1. la 1-amino-3-cyclopropyl-1-(2-méthylthio-4-chlorophényl)-1-propène-3-one ;
2. la 1-amino-3-cyclopropyl-1-(2-méthylthio-4-trifluorométhylphényl)-1-propène-3-one ;
3. la 1-amino-3-cyclopropyl-1-(2-méthylthio-4-bromophényl)-1-propène-3-one ;
4. la 1-amino-3-cyclopropyl-1-(2-méthylthio-3,4-dichlorophényl)-1-propène-3-one ;
5. la 1-amino-3-cyclopropyl-1-(2-méthyl-4-méthylthipphényl)-1-propène-3-one ;
6. la 1-amino-3-cyclopropyl- 1 -(2-méthylthio-3-chloro4-bromophényl)-1-propène-3-one ;
7. la 1-amino-3-cyclopropyl-1-(2-méthylthio-3-éthoxy-4-chlorophényl)-1-propène-3-one ;
8. la 1-amino-3-cyclopropyl-1-(2-méthylthio-3,4-dibromophényl)-1-propène-3-one.

II - Dans son second objet, la présente invention concerne un procédé de préparation des β-aminovinylcétones de formule (VI) ou de mélanges à base d'aminovinylcétones qui est caractérisé en ce qu'il comprend la réaction de condensation d'une méthylorganocétone de formule : 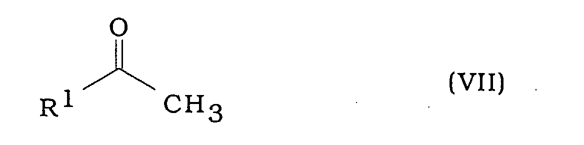 dans laquelle R¹ possède l'une des définitions générale ou préférées indiquées ci-avant à propos de la formule (VI), avec un nitrile aromatique de formule : 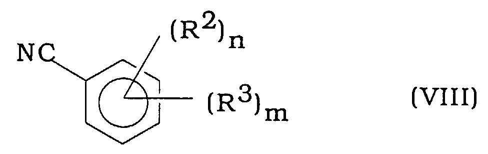 dans laquelle les symboles R², R³, n et m possèdent l'une des définitions générales ou préférées indiquées ci-avant à propos de la formule (VI), ladite réaction de condensation étant conduite sous atmosphère inerte en opérant un chauffage prolongé, en milieu solvant inerte, en présence d'une base forte prise dans le groupe formé par :
(i) les alcoolates dérivés de métaux alcalins et de monoalcools primaires dans la structure desquels le reste associé au groupe CH₂OH est un groupe à chaîne ramifiée dans lequel l'atome de carbone directement lié au groupe CH₂OH est lui-même lié à 2 ou 3 autres atomes de carbones, ces autres atomes de carbone appartenant à des radicaux, identiques ou différents, choisis chacun parmi un radical alkyle linéaire ou ramifié ayant jusqu'à 3 atomes de carbone et un radical phényle ;
(2i) les alcoolates dérivés de métaux alcalins et de monoalcools secondaires ou tertiaires dans la structure desquels chacun des 2 restes, identiques ou différents, associés au groupe CHOH dans le cas des alcools secondaires ou chacun des 3 restes, identiques ou différents, associés au groupe COH dans le cas des alcools tertiaires, consiste dans un radical choisi parmi un radical alkyle linéaire ou ramifié ayant jusqu'à 4 atomes de carbone et un radical phényle ; et
(3i) les solutions des alcoolates (i) ou (2i) dans les monoalcools qui leur ont donné naissance.

La réaction de condensation est effectuée généralement sous une légère surpression d'un gaz inerte, comme par exemple l'azote ou l'argon, et au sein d'un solvant inerte ou d'un mélange de solvants inertes.

Les solvants préférés sont des solvants polaires, aprotiques, non miscibles à l'eau, comme par exemple le tétrahydrofuranne, le dioxanne, le diéthyléther, le méthyltertiobutyléther, le benzène, le chlorobenzène, le toluène.

La réaction est effectuée à une température se situant dans l'intervalle allant de 30°C à 120°C ; de préférence, la température choisie est celle, comprise dans l'intervalle précité, qui correspond à la température de reflux du mélange réactionnel.

La durée de la réaction peut varier, selon la température choisie, entre par exemple 30 minutes et 5 heures.

La réaction est effectuée de préférence avec les proportions molaires suivantes d'ingrédients :
- nitrile aromatique de formule (VIII) : 1 mole,
- R¹ méthylcétone de formule (VII) : 1 à 4 moles et de préférence 1,3 à 2 moles,
- alcoolate : 1 à 4 moles et de préférence 1,3 à 2 moles.

S'agissant de la base forte mise en oeuvre, on peut faire appel par exemple aux alcoolates de sodium ou de potassium dérivés des alcools suivants :
* alcool isobutylique (CH₃)₂ CH-CH₂OH,
* alcool isopropylique (CH₃)₂ -CHOH,
* alcool sec-butylique CH₃(C₂H₅)-CHOH,
* alcool tertiobutylique (CH₃)₃-COH,
* alcool diméthylphenylméthylique (CH₃)₂(C₆H₅)-COH,
* alcool triphénylméthylique (C₆H₅)₃-COH,
ou aux solutions de ces alcoolates dans les alcools précités.

Les meilleurs résultats sont obtenus en opérant la réaction de condensation en présence d'une base forte prise dans le groupe formé par :
(4i) les alcoolates dérivés de métaux alcalins et de monoalcools tertiaires dans la structure desquels chacun des 3 restes, identiques ou différents, associés au groupe COH est un radical choisi parmi un radical alkyle linéaire ayant 1 ou 2 atomes de carbone et un radical phényle, comme par exemple les alcoolates de sodium et de potassium dérivés des alcools de formule suivantes : (CH₃)₃-COH, (CH₃)₂ (C₆H₅)-COH et (C₆H₅)₃-COH ; et
(5i) les solutions des alcoolates (4i) dans les monoalcools qui leur ont donné naissance.

La préparation de l'alcoolate peut être réalisée de différentes manières : on peut préparer l'alcoolate par dissolution du métal alcalin dans l'alcool correspondant ; on peut encore préparer l'alcoolate en faisant réagir un méthylate alcalin avec l'alcool correspondant ; une autre façon d'opérer consiste encore à faire réagir un hydrure alcalin avec l'alcool correspondant. La préparation de l'alcoolate peut être réalisée en dehors du milieu de la réaction de condensation ou éventuellement in situ. Habituellement, cette préparation est faite, de manière connue en soi, en utilisant 1 à 5 moles et de préférence 1,1 à 4 moles de l'alcool correspondant pour 1 mole du réactif alcalin (métal alcalin, méthylate alcalin, hydrure alcalin). L'alcoolate formé peut être utilisé soit à l'état pur, soit sous forme de solution dans l'alcool n'ayant pas réagi qui lui a donné naissance, lorsque ce dernier est utilisé en excès. Dans le présent mémoire, quand on parle de "solutions des alcoolates dans les monoalcools qui leur ont donné naissance", on entend définir des solutions qui peuvent renfermer jusqu'à 4 moles d'alcool et, de préférence, entre 0,1 et 3 moles d'alcool pour 1 mole de base forte alcoolate.

Quand tout le nitrile de formule (VIII) a réagi, une manière de terminer la réaction peut consister à refroidir le milieu réactionnel, à l'amener à pH neutre par addition d'une quantité déterminée d'un hydracide ou d'un oxyacide minéral et à ajouter de l'eau, en quantité suffisante pour dissoudre les sels formés ; avantageusement, l'acide et l'eau peuvent être introduits ensemble sous forme d'une solution aqueuse diluée de l'acide.

Lorsque la réaction de condensation est conduite dans un milieu solvant non miscible à l'eau, on obtient, à l'issue du protocole de traitement qui vient d'être décrit, un milieu biphasique hydro-organique. La phase organique qui renferme l'aminovinylcétone formée est séparée, par exemple par décantation, puis concentrée par élimination du (ou des) solvant(s).

Le produit résiduel ainsi obtenu après élimination du (ou des) solvant(s) est constitué essentiellement de la β-aminovinylcétone de formule (VI). Par l'expression "constitué essentiellement", on veut signifier que la β-aminovinylcétone peut dans certains cas se trouver dans le produit résiduel sous forme de mélange avec une quantité minoritaire de composé(s) secondaire(s) consistant dans :
* une pyrimidine de formule générale : 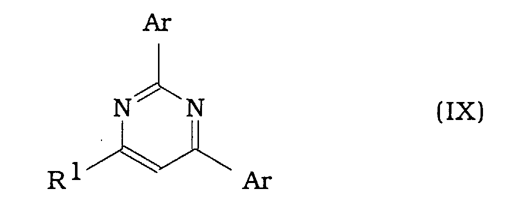
* et/ou un amide de formule générale : 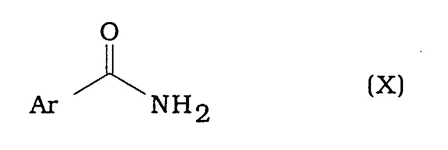
formules dans lesquelles les symboles Ar, qui sont identiques entre eux et représentent le groupe : 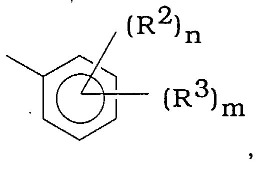 et le symbole R¹ possèdent les définitions générales ou préférées indiquées ci-avant à propos de l'aminovinylcétone de formule (VI).

Dans le présent mémoire, on désignera par l'expression "mélange à base d'aminovinylcétone", le produit résiduel consistant dans le mélange de β-aminovinylcétone de formule (VI) avec une quantité minoritaire de composé(s) secondaire(s) de formules (IX) et/ou (X). Les quantités de composé(s) secondaire(s), exprimées en % en mole de composé(s) secondaire(s) dans le mélange à base d'aminovinylcétone, peuvent être au plus égales à :
- pyrimidine de formule (IX) : 14 %,
- amide de formule (X) : 35 %.

Quand le produit résiduel est sous forme d'un mélange à base d'aminovinylcétone, il est possible d'obtenir une β-aminovinylcétone à l'état pur en soumettant le mélange à une opération de recristallisation dans un solvant approprié tel que par exemple le toluène ou des mélanges toluène/cyclohexane.

Lorsque la réaction de condensation est conduite dans un milieu solvant miscible à l'eau, on peut isoler la β-aminovinylcétone ou un mélange à base d'aminovinylcétone en soumettant le milieu réactionnel, après ajout d'acide et d'eau, par exemple à une opération d'extraction avec un tiers solvant approprié, éventuellement en ayant éliminé au préalable tout ou partie du (ou des) solvant(s) réactionnel(s).

III - Dans son troisième objet, la présente invention concerne l'utilisation des β-aminovinylcétones de formule (VI) ou de mélanges à base d'aminovinylcétones dans la préparation de β-dicétones de formule générale : 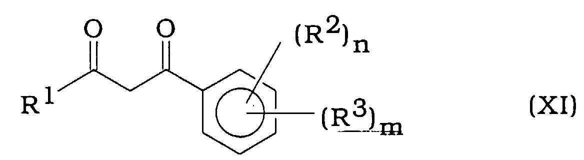 dans laquelle les symboles R¹, R², R³, n et m possèdent l'une des définitions générales ou préférées indiquées ci-avant à propos de la formule (VI), ladite utilisation étant caractérisée en ce qu'elle comprend l'opération de soumettre une β-aminovinylcétone de formule (VI) ou un mélange à base d'aminovinylcétone à une réaction d'hydrolyse en opérant, éventuellement au sein d'un milieu solvant inerte, en présence d'eau et d'un acide fort minéral ou organique.

La réaction d'hydrolyse peut être conduite sans solvant ou en milieu solvant par addition alors aux réactifs de départ d'un solvant inerte ou mélange de solvants inertes.

Quand on opère en milieu solvant, et cela correspond à un mode opératoire préféré, on utilise avantageusement un milieu solvant qui est identique à celui mis en oeuvre dans le cadre du procédé conforme à la présente invention prise dans son second objet, et en particulier un milieu solvant qui, non seulement est identique, mais encore est polaire, aprotique et non miscible à l'eau. Un mode opératoire encore plus préféré consiste alors à engager la β-aminovinylcétone ou le mélange à base d'aminovinylcétone sous la forme de la phase organique telle qu'elle est obtenue, après séparation de la phase aqueuse, à partir du milieu biphasique hydro-organique qui est obtenu à l'issue du protocole de traitement du milieu réactionnel du procédé conforme à la présente invention prise dans son second objet ; ce mode opératoire (encore plus préféré) est particulièrement intéressant car il permet d'enchaîner les réactions de condensation (second objet de l'invention) et d'hydrolyse (troisième objet de l'invention) en travaillant dans le même appareillage, sans avoir besoin d'isoler la β-aminovinylcétone à l'issue de la réaction de condensation et sans avoir besoin de changer fondamentalement la composition du milieu réactionnel pour passer de la réaction de condensation à la réaction d'hydrolyse.

La réaction d'hydrolyse est effectuée à une température se situant dans l'intervalle allant de 40°C à 120°C ; quand on opère en milieu solvant, la température choisie est alors de préférence celle, comprise dans l'intervalle précité, qui correspond à la température de reflux du mélange réactionnel.

La durée de la réaction peut varier, selon la température choisie, entre par exemple 30 minutes et 5 heures.

La réaction est effectuée de préférence avec les proportions suivantes d'ingrédients:
- β-aminovinylcétone de formule (VI) : 1 mole,
- Eau : 1 à 20 moles et de préférence 1,5-10 moles,
- Acide fort : 1 à 6 ions H⁺ et de préférence 1,1 à 4 ions H⁺.

Par acide fort, minéral ou organique, on entend notamment un mono-ou polyacide oxygéné ou non dont l'une au moins des fonctions acides (quand il y en a plusieurs) possède une constante d'ionisation dans l'eau, pKa, inférieure ou égale à 3. Comme acides de ce type on peut citer, parmi les acides minéraux, les acides chlorhydrique, sulfurique, orthophosphorique, pyrophosphorique. Parmi les acides organiques, on peut citer : les acides organosulfoniques, en particulier paratoluénesulfonique, méthanesulfonique, naphtalènesulfonique ; les acides organophosphoniques, en particulier les acides monoalkyl- ou monoarylphosphoniques tels que méthylphosphonique ou benzènephosphonique ; les acides forts polycarboxyliques halogénés, tels que les acides dihalogéno- et trihalogéno- (notamment chloro et fluoro) acétique ou propioniques. Dans l'invention, on préfère utiliser comme acide fort les mono- ou polyacides minéraux oxygénés ou non et plus particulièrement les acides chlorhydrique et sulfurique.

De manière avantageuse, l'eau et l'acide fort, qui sont nécessaires à la réaction, peuvent être introduits ensemble dans le milieu sous forme d'une solution aqueuse d'acide dont la normalité et la quantité sont déterminées de manière à apporter la quantité d'eau et le nombre d'ions H⁺ qui sont requis.

Quand toute la β-aminovinylcétone a réagi, la réaction est terminée en refroidissant le milieu réactionnel et en procédant ensuite au traitement, de manière connue en soi, de ce milieu pour en isoler la β-dicétone formée de formule (XI).

Par exemple, lorsque la réaction d'hydrolyse est conduite dans un milieu solvant non miscible à l'eau, on est alors en présence d'un milieu réactionnel biphasique hydro-organique, et ce traitement peut consister alors à séparer la phase organique qui renferme la β-dicétone formée, par exemple par décantation, et à soumettre ensuite ladite phase organique à une concentration par élimination du (ou des) solvant(s) réactionnel(s).

Lorsque la réaction d'hydrolyse est conduite en partant d'un mélange à base d'aminovinylcétone, on retrouve généralement dans la phase organique finale, à côté de la β-dicétone, le (ou les) composé(s) secondaire(s) pyrimidine et/ou amide (qui n'a pas été substantiellement modifié dans la réaction hydrolyse) dont on a parlé ci-avant.

Après élimination du (ou des) solvant(s), la β-dicétone obtenue peut être purifiée, au besoin, par recristallisation de manière connue en soi.

Les exemples suivants donnés à titre non limitatif illustrent l'invention et montrent comment elle peut être mise en pratique.

### EXEMPLE 1

### Exemple de préparation d'une β-aminovinylcétone selon le procédé de l'invention prise dans son second objet.

L'appareillage utilisé consiste dans 1 ballon tricol en verre de 100 ml équipé d'un thermomètre, d'un réfrigérant ascendant, d'un système de chauffage, d'un système d'agitation et d'un système de circulation d'argon sous une légère surpression.

On ajoute successivement dans le réacteur à température ambiante (25°C):
- 10,9 g (0,050 mole) de 1-cyano -2-méthylthio-4-trifluorométhylbenzène,
- 50 cm³ de méthyltertiobutyléther,
- 7,5 cm³ (0,075 mole) de cyclopropylméthylcétone, et
- 7,2 g (0,075 mole) de tertiobutylate de sodium à l'état pur.

Ce mélange est agité et chauffé au reflux (57°C) pendant 3 heures 30 minutes.

Après refroidissement à température ambiante, on ajoute dans le milieu réactionnel 25 cm³ d'une solution aqueuse d'HCl 1N qui amène le pH à une valeur neutre et conduit à l'obtention d'un milieu biphasique hydro-organique.

La phase aqueuse et la phase organique sont séparées. La phase organique est ensuite extraite avec 25 cm³ d'eau, puis elle est concentrée par élimination du solvant par distillation sous pression réduite.

Le produit résiduel ainsi obtenu est analysé par chromatographie liquide, haute performance. Les résultats de cette analyse sont les suivants :
1) taux de transformation molaire du nitrile de départ : 100 %.
2) il s'est formé un mélange des 3 composés suivants :
   2.1. β-aminovinylcétone de formule : 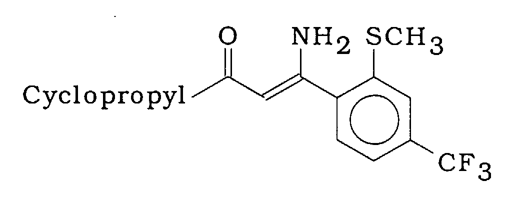 avec un rendement molaire, par rapport au nitrile ayant réagi, égal à 92 % ;
   2.2. Pyrimidine de formule : 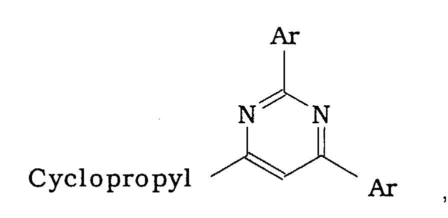 avec 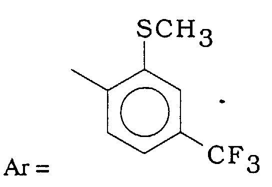 avec un rendement molaire, par rapport au nitrile ayant réagi, égal à 4 % ; et
   2.3. Amide de formule : 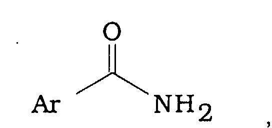 avec 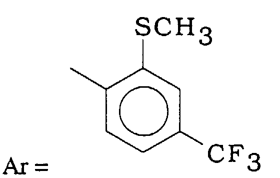 avec un rendement molaire, par rapport au nitrile ayant réagi, égal à 4 %.

La β-aminovinylcétone à l'état pur a été obtenue par recristallisation du mélange précité dans 50 cm³ de toluène. Les analyses RMN, IR et le spectre de masse sont en accord avec la structure présentée ci-avant :
- RMN (¹H, CDCl₃) : 0,75 - 0,98 ppm (m, 4 H) ; 1,71 ppm (m, 1H) ; 2,44 ppm (s, 3H) ; 5,0 ppm (s, 1H) ; 5,28 ppm (s, 1H) ; 7,38 ppm (m, 3H) ; 9,65 ppm (s, 1H) ;
- Spectre IR : 3 413 - 3 254 cm⁻¹ (NH₂) ; 1613 cm⁻¹ (C = O) ; 1320 - 1140 cm⁻¹ (CF₃) ; 2924 cm⁻¹ (SCH₃) ;
- spectre de masse (appareil : VG ZAB 2 S F ; source : EICI ; M/ΔM = 2000) : M = 301 g.

### EXEMPLE 2

### Exemple de préparation d'une β-dicétone selon une modalité d'utilisation conforme à l'invention prise dans son troisième objet.

L'appareillage utilisé est celui déjà décrit dans l'exemple 1.

On reproduit l'exemple 1 exactement comme indiqué, mais on s'arrête au stade de l'obtention du milieu biphasique hydro-organique et on sépare la phase aqueuse de la phase organique qui est laissée dans le réacteur utilisé. Cette phase organique renferme les trois composés 2.1., 2.2. et 2.3. qui ont été décrits ci-avant dans l'exemple 1.

A la phase organique est ensuite ajoutée 10 cm³ d'une solution aqueuse d'acide sulfurique 10 N. On forme de cette manière un nouveau milieu biphasique hydro-organique qui est agité et chauffé à 60°C pendant 3 heures.

Au bout de ce temps, après refroidissement à température ambiante (25°C), les deux phases sont séparées. La phase organique est extraite avec 20 cm³ d'une solution aqueuse de NaHCO₃ renfermant 5 g de bicarbonate par litre, puis avec 20 cm³ d'eau. Le solvant de la phase organique est ensuite éliminé. Le produit résiduel obtenu est recristallisé dans 40 cm³ de méthanol et il conduit de cette manière à l'obtention de la β-dicétone, à l'état pur, de formule :

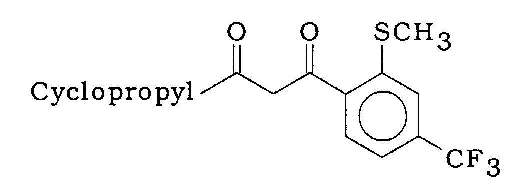

Les analyses RMN et par spectrométrie de masse sont en accord avec la structure indiquée ci-avant :
- RMN(¹H, CDCl₃) :
   * forme énol (90 % en mole) : 0,96 - 1,17 ppm (mn 4 H) ; 1,09 ppm (m, 1H) ; 2,45 ppm (s, 3H) ; 6,03 ppm (s, 1 H pour = CH - CO) ; 7,35 - 7,56 ppm (m, 3H) ; > 11 ppm (s, 1H) ;
   * forme cétone (10 % en mole) : 4,2 ppm (s, 2H pour CO-CH₂-CO) ;
- spectre de masse : M = 302 g ;
- à noter encore : PF = 60°C.

## Revendications

1. β-aminovinylcétones de formule générale : 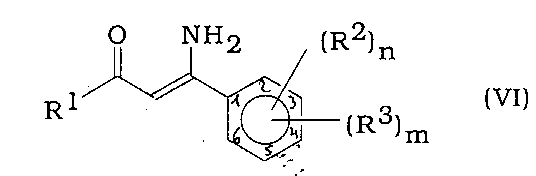 dans laquelle :
- R¹ représente :
* un groupe alcényle ou alcynyle à chaîne droite ou ramifiée contenant jusqu'à 6 atomes de carbone qui est facultativement substitué avec un ou plusieurs atome(s) d'halogène et dont l'insaturation n'est pas conjugée avec la double liaison cétonique;
* un groupe cycloalkyle contenant 3 à 6 atomes de carbone, éventuellement substitué avec un ou plusieurs groupe(s) R⁴ ou OR⁴ ou un ou plusieurs atome(s) d'halogène ;
* un groupe cycloalcényle contenant 4 à 6 atomes de carbone, éventuellement substitué avec un ou plusieurs groupe(s) R⁴ ou OR⁴ ou un ou plusieurs atome(s) d'halogène, dans lequel l'insaturation éthylénique n'est pas conjuguée avec la double liaison cétonique ;
* un groupe de formule (R⁵ 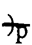 phényl(̵CR⁶R⁷)_{q}- ;
* R⁴ étant un groupe alkyle à chaîne droite ou ramifiée contenant jusqu'à 4 atomes de carbone;
* R⁵ étant un groupe alkyle à chaîne droite ou ramifiée contenant jusqu'à 4 atomes de carbone ou un atome d'halogène (F, Cl, Br, I) ;
* R⁶ et R⁷, qui peuvent être identiques ou différents, représentant chacun un groupe alkyle à chaîne droite ou ramifiée contenant jusqu'à 4 atomes de carbone ;
* p et q étant des nombres entiers, qui peuvent être identiques ou différents, allant de 0 à 3 ;
- R² représente un substituant attracteur d'électrons choisi dans le groupe formé par :
* un groupe nitro ;
* un groupe acyle COR⁴ ;
* un groupe COOR⁸ ;
* un groupe alkylthio SR⁴ ;
* un groupe carbamoyle CONR⁸R⁹ ou thiocarbamoyle CSNR⁸R⁹ ;
* un groupe halogénoalkyle ou halogénoalkoxy linéaire ou ramifié ayant jusqu'à 4 atomes de carbone;
* R⁴ ayant les définitions générales indiquées ci-avant ;
* R⁸ et R⁹, qui peuvent être identiques ou différents, représentant chacun un atome d'hydrogène ou un groupe alkyle à chaîne droite ou ramifiée contenant jusqu'à 4 atomes de carbone ;
- R³ représente :
* un groupe alkyle ou alkoxy à chaîne droite ou ramifiée contenant jusqu'à 4 atomes de carbone, le groupe alkyle pouvant éventuellement être substitué avec un groupe OR⁴ ;
* un atome d'halogène ;
* un groupe de formule O-(-CH₂)ᵣ-OR⁴ ;
* R⁴ possédant la définition générale indiquée ci-avant et r étant un nombre entier allant de 1 à 4 ;
- le symbole n représente un nombre entier allant de 0 à 2 et le symbole m représente un nombre entier allant de 0 à 3 ;
- avec les réserves a) et b) suivantes :
a) R⁵ est différent de l'atome de chlore quand R³ est l'atome de chlore et q = 0,
b) quand n, m et q sont tous égaux à zéro, alors p est différent de zéro.

2. β-aminovinylcétones selon la revendication 1, caractérisées en ce qu'elles répondent à la formule (VI) dans laquelle le symbole n représente un nombre allant de 1 à 2 et le symbole m représente un nombre allant de 0 à 3.

3. β-aminovinylcétones selon la revendication 2, caractérisées en ce que ce sont des composés dans la structure desquels :
- R¹ représente un groupe cycloalkyle contenant 3 à 6 atomes de carbone, éventuellement substitué avec un ou plusieurs groupe(s) R⁴ ou OR⁴ ;
- R² représente un groupe alkylthio SR⁴, un groupe halogénoalkyle ou halogénoalkoxy linéaire ou ramifié ayant jusqu'à 4 atomes de carbone;
- R³ représente un groupe alkyle ou alkoxy à chaîne droite ou ramifiée contenant jusqu'à 4 atomes de carbone ou un atome d'halogène.

4. β-aminovinylcétones selon la revendication 3, caractérisées en ce que ce sont des composés dans la structure desquels :
- le symbole n représente un nombre entier égale à 1 ou 2 et le symbole m représente un nombre entier allant de 0 à 2, avec la condition supplémentaire selon laquelle la somme m + n est au plus égale à 3 ;
- le (ou l'un des) groupe(s) représenté(s) par (R²)ₙ est en position 2 sur le noyau phényle tandis que le (ou les) éventuelle(s) autre(s) groupe(s) représenté(s) par (R²)ₙ et/ou (R³)ₘ sont en position 3 et/ou 4 sur le noyau phényle.

5. β-aminovinylcétones selon la revendication 4, caractérisées en ce que ce sont des composés dans la structure desquels :
- R¹ représente un groupe cyclopropyle ou 1-méthylcyclopropyle ;
- R² représente un groupe méthylthio ou éthylthio ou un groupe trifluorométhyle ou trifluorométhoxy ;
- R³ représente un groupe méthyle, éthyle, méthoxy ou éthoxy ou un atome de chlore, de brome ou de fluor.

6. Procédé de préparation des β-aminovinylcétones de formule (VI) selon l'une quelconque des revendications 1 à 5, caractérisé en ce qu'il comprend la réaction de condensation d'une méthylorganocétone de formule : 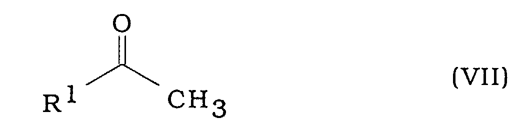 dans laquelle R¹ possède l'une des définitions générale ou préférées indiquées ci-avant à propos de la formule (VI), avec un nitrile aromatique de formule : 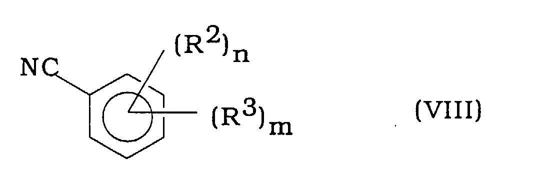 dans laquelle les symboles R², R³, n et m possèdent l'une des définitions générales ou préférées indiquées ci-avant à propos de la formule (VI), ladite réaction de condensation étant conduite sous atmosphère inerte en opérant un chauffage prolongé, en milieu solvant inerte, en présence d'une base forte prise dans le groupe formé par :
(i) les alcoolates dérivés de métaux alcalins et de monoalcools primaires dans la structure desquels le reste associé au groupe CH₂OH est un groupe à chaîne ramifiée dans lequel l'atome de carbone directement lié au groupe CH₂OH est lui-même lié à 2 ou 3 autres atomes de carbones, ces autres atomes de carbone appartenant à des radicaux, identiques ou différents, choisis chacun parmi un radical alkyle linéaire ou ramifié ayant jusqu'à 3 atomes de carbone et un radical phényle ;
(2i) les alcoolates dérivés de métaux alcalins et de monoalcools secondaires ou tertiaires dans la structure desquels chacun des 2 restes, identiques ou différents, associés au groupe CHOH dans le cas des alcools secondaires ou chacun des 3 restes, identiques ou différents, associés au groupe COH dans le cas des alcools tertiaires, consiste dans un radical choisi parmi un radical alkyle linéaire ou ramifié ayant jusqu'à 4 atomes de carbone et un radical phényle ; et
(3i) les solutions des alcoolates (i) ou (2i) dans les monoalcools qui leur ont donné naissance,
et en ce qu'il produit éventuellement des mélanges à base d'aminovinylcétones comprenant, outre le composé VI, notamment une pyrimidine de formule générale : 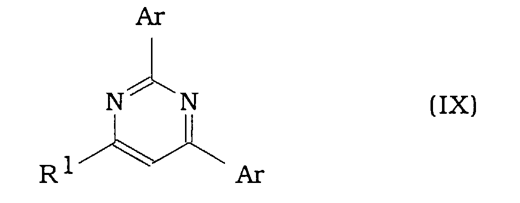
- dans laquelles les symboles Ar, qui sont identiques entre eux et représentent le groupe : 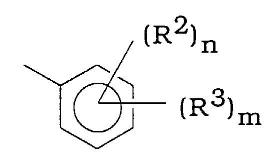 et le symbole R¹ possèdent les définitions générales ou préférées indiquées ci-avant, à propos de l'aminovinylcétone de formule (VI), dans l'une quelconque des revendications 1 à 5,
- avec les réserves suivantes pour ce composé IX :
* (a) quand n = 0 et m = 0 ou (b) quand n = 0 et m = 1 ou 2 et R³ représente un groupe alkyle, alkoxy ou un atome d'halogène, R¹ dans chaque cas (a) ou (b) est alors différent d'un groupe alkyle ou d'un groupe de formule (R⁵-)ₚ -phényl- (-CR⁶R⁷)_{q}- où q = 0 ;
* (c) quand n = 1 et m = 0 et R² représente NO₂, R¹ est alors différent d'un groupe de formule (R⁵-)ₚ -phényl- (-CR⁶R⁷)_{q}- où q = 0.

7. Procédé selon la revendication 6, caractérisé en ce que le milieu solvant consiste dans un solvant inerte ou un mélange de solvants inertes choisi(s) parmi les solvants polaires, aprotiques, non miscibles à l'eau.

8. Procédé selon l'une des revendications 6 et 7, caractérisé en ce que la réaction est effectuée avec les proportions molaires suivantes d'ingrédients :
- nitrile aromatique de formule (VIII) : 1 mole,
- R¹ méthylcétone de formule (VII) : 1 à 4 moles,
- alcoolate : 1 à 4 moles.

9. Procédé selon l'une quelconque des revendications 6 à 8, caractérisé en ce que l'on effectue la réaction de condensation en présence d'une base forte prise dans le groupe formé par :
(4i) les alcoolates dérivés de métaux alcalins et de monoalcools tertiaires dans la structure desquels chacun des 3 restes, identiques ou différents, associés au groupe COH est un radical choisi parmi un radical alkyle linéaire ayant 1 ou 2 atomes de carbone et un radical phényle ; et
(5i) les solutions des alcoolates (4i) dans les monoalcools qui leur ont donné naissance.

10. Procédé selon la revendication 9, caractérisé en ce que les alcoolates visés sont les alcoolates de sodium et de potassium dérivés des alcools de formule suivantes : (CH₃)₃-COH, (CH₃)₂ (C₆H₅)-COH et (C₆H₅)₃-COH.

11. Procédé selon l'une quelconque des revendications 6 à 10, caractérisé en ce que la réaction est effectuée à une température se situant dans l'intervalle allant de 30°C à 120°C.

12. Utilisation des β-aminovinylcétones de formule (VI) selon l'une quelconque des revendications 1 à 5 ou de mélanges à base d'aminovinylcétones dans la préparation de β-dicétones de formule générale : 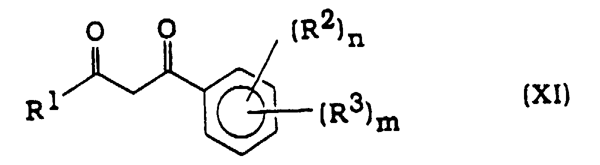 dans laquelle les symboles R¹, R², R³, n et m possèdent l'une des définitions générales ou préférées indiquées ci-avant, à propos de la formule (VI), dans l'une quelconque des revendications 1 à 5, ladite utilisation étant caractérisée en ce qu'elle comprend l'opération de soumettre une β-aminovinylcétone de formule (VI) ou un mélange à base d'aminovinylcétone à une réaction d'hydrolyse en opérant, éventuellement au sein d'un milieu solvant inerte, en présence d'eau et d'un acide fort minéral ou organique.

13. Utilisation selon la revendication 12, caractérisée en ce que l'on opère dans un milieu solvant qui est identique à celui mis en oeuvre dans le cadre du procédé de préparation, selon la revendication 6, des β-aminovinylcétones de formule (VI).

14. Utilisation selon la revendication 13, caractérisée en ce que le milieu solvant consiste dans un solvant inerte ou un mélange de solvants inertes choisi(s) parmi les solvants polaires, aprotiques, non miscibles à l'eau.

15. Utilisation selon l'une quelconque des revendications 12 à 14, caractérisée en ce que la réaction est effectuée avec les proportions suivantes d'ingrédients :
- β-aminovinylcétone de formule (VI) : 1 mole,
- Eau : 1 à 20 moles,
- Acide fort : 1 à 6 ions H⁺.

16. Utilisation selon l'une quelconque des revendications 12 à 15, caractérisée en ce que l'acide fort a une constante d'ionisation dans l'eau, pKa, inférieure ou égale à 3.

17. Utilisation selon la revendication 16, caractérisé en ce que l'acide est choisi dans le groupe constitué par les acides minéraux, les acides organosulfoniques, les acides organophosphoniques et les acides carboxyliques halogénés.

18. Utilisation selon l'une quelconque des revendications 12 à 17, caractérisée en ce que la réaction d'hydrolyse est effectuée à une température se situant dans l'intervalle allant de 40°C à 120°C.

## Patentansprüche

1. β-Aminovinylketone der allgemeinen Formel: 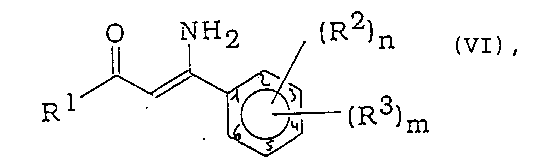 worin bedeuten:
- R¹
• eine geradkettige oder verzweigte Alkenyl- oder Alkinylgruppe mit bis zu 6 Kohlenstoffatomen, die ggf. mit einem oder mehreren Halogenatomen substituiert sind und deren ungesättigte Gruppe nicht mit der Keton-Doppelbindung konjugiert ist;
• eine Cycloalkylgruppe mit 3 bis 6 Kohlenstoffatomen, die ggf. mit einer oder mehreren Gruppen R⁴ oder OR⁴ oder einem oder mehreren Halogenatomen substituiert ist;
• eine Cycloalkenylgruppe mit 4 bis 6 Kohlenstoffatomen, die ggf. mit einer oder mehreren Gruppen R⁴ oder OR⁴ oder einem oder mehreren Halogenatomen substituiert ist, wobei die ethylenisch ungesättigte Gruppe nicht mit der Keton-Doppelbindung konjugiert ist;
• eine Gruppe der Formel (R⁵-)ₚ-Phenyl-(-CR⁶R⁷)_{q}-; wobei
R⁴ eine geradkettige oder verzweigte Alkylgruppe mit bis zu 4 Kohlenstoffatomen ist;
R⁵ eine geradkettige oder verzweigte Alkylgruppe mit bis zu 4 Kohlenstoffatomen oder ein Halogenatom (F, Cl, Br, I) ist;
R⁶ und R⁷, die identisch oder voneinander verschieden sein können, jeweils eine geradkettige oder verzweigte Alkylgruppe mit bis zu 4 Kohlenstoffatomen bedeuten;
und
p und q, die identisch oder voneinander verschieden sein können, 0, 1, 2 oder 3 bedeuten;
- R² einen elektronenanziehenden Substituenten, der ausgewählt ist unter:
• der Nitro-Gruppe;
• einer Acylgruppe COR⁴;
• einer Gruppe COOR⁸;
• einer Alkylthiogruppe SR⁴;
• einer Carbamoylgruppe CONR⁸R⁹ oder Thiocarbamoylgruppe CSNR⁸R⁹;
• einer geradkettigen oder verzweigten Halogenalkyl- oder Halogenalkoxygruppe mit bis zu 4 Kohlenstoffatomen;
wobei
R⁴ den oben angegebenen allgemeinen Definitionen entspricht;
und
R⁸ und R⁹, die identisch oder voneinander verschieden sein können, jeweils ein Wasserstoffatom oder eine geradkettige oder verzweigte Alkylgruppe mit bis 4 Kohlenstoffatomen bedeuten;
- R³:
• eine geradkettige oder verzweigte Alkyl- oder Alkoxygruppe mit bis zu 4 Kohlenstoffatomen, wobei die Alkylgruppe ggf. mit einer Gruppe OR⁴ substituiert sein kann;
• ein Halogenatom;
• eine Gruppe der Formel O-(-CH₂)ᵣ-OR⁴;
wobei R⁴ die oben angegebene Bedeutung aufweist und r eine ganze Zahl im Bereich von 1 bis 4 ist;
- n 0, 1 oder 2 und m 0, 1, 2 oder 3;
- mit den folgenden Maßgaben a) und b):
a) R⁵ ist von einem Chloratom verschieden, wenn R³ ein Chloratom ist und q Null ist, und
b) p ist von Null verschieden, wenn n, m und q gleichzeitig Null bedeuten.

2. β-Aminovinylketone nach Anspruch 1, dadurch gekennzeichnet, daß sie der Formel VI entsprechen, worin n eine Zahl von 1 bis 2 und m eine Zahl von 0 bis 3 bedeuten.

3. β-Aminovinylketone nach Anspruch 2, dadurch gekennzeichnet, daß sie folgende Struktur aufweisen:
- R¹ bedeutet eine Cycloalkylgruppe mit 3 bis 6 Kohlenstoffatomen, die ggf. mit einer oder mehreren Gruppen R⁴ oder OR⁴ substituiert ist;
- R² bedeutet eine Alkylthiogruppe SR⁴, eine geradkettige oder verzweigte Halogenalkyl- oder Halogenalkoxygruppe mit bis zu 4 Kohlenstoffatomen;
- R³ bedeutet eine geradkettige oder verzweigte Alkyl- oder Alkoxygruppe mit bis zu 4 Kohlenstoffatomen oder ein Halogenatom.

4. β-Aminovinylketone nach Anspruch 3, dadurch gekennzeichnet, daß sie folgende Struktur aufweisen:
- n bedeutet eine ganze Zahl 1 oder 2 und m 0, 1 oder 2, mit der zusätzlichen Maßgabe, daß die Summe m + n höchstens 3 ist;
- sich die durch (R²)ₙ dargestellte Gruppe oder eine der durch (R²)ₙ dargestellten Gruppen in 2-Stellung des Phenylrings befindet, wohingegen sich die ggf. vorhandene(n) weitere(n) Gruppe(n), die durch (R²)ₙ und/oder (R³)ₘ dargestellt ist (sind), in 3- und/oder 4-Stellung des Phenylrings befinden.

5. β-Aminovinylketone nach Anspruch 4, dadurch gekennzeichnet, daß sie folgende Struktur aufweisen:
- R¹ bedeutet Cyclopropyl oder 1-Methylcyclopropyl;
- R² bedeutet Methylthio, Ethylthio, Trifluormethyl oder Trifluormethoxy;
- R³ bedeutet Methyl, Ethyl, Methoxy, Ethoxy, Chlor, Brom oder Fluor.

6. Verfahren zur Herstellung von β-Aminovinylketonen der Formel VI nach einem der Ansprüche 1 bis 5, dadurch gekennzeichnet, daß es die Kondensation eines Methylorganoketons der Formel 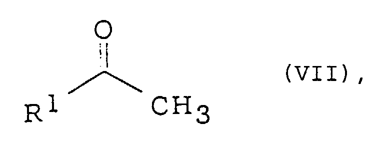 worin R¹ eine der für die Formel VI angegebenen allgemeinen oder bevorzugten Bedeutungen aufweist,
mit einem aromatischen Nitril der folgenden Formel umfaßt: 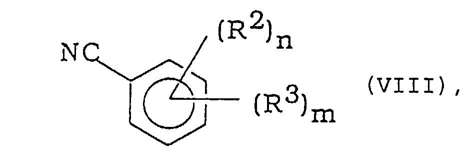 worin die Symbole R², R³, n und m eine der für die Formel VI angegebenen allgemeinen oder bevorzugten Definitionen aufweisen, wobei die Kondensation in einer inerten Atmosphäre unter ausgiebigem Erwärmen in einem inerten Lösungsmittelmedium in Gegenwart einer starken Base durchgeführt wird, welche ausgewählt ist unter:
(i) den Alkoholaten, die von Alkalimetallen und primären Monoalkoholen abgeleitet sind, wobei in der Struktur des Monoalkohols der an die Gruppe CH₂OH gebundene Rest eine verzweigte Gruppe ist, worin das direkt an die Gruppe CH₂OH gebundene Kohlenstoffatom selbst an zwei oder drei andere Kohlenstoffatome gebunden ist, wobei die anderen Kohlenstoffatome zu Gruppen gehören, die identisch oder voneinander verschieden sind und unter geradkettigen oder verzweigten Alkylgruppen mit bis zu 3 Kohlenstoffatomen oder Phenyl ausgewählt sind;
(2i) den Alkoholaten, die von Alkalimetallen und sekundären oder tertiären Monoalkoholen abgeleitet sind, wobei in der Struktur der Monoalkohole im Falle der sekundären Alkohole die beiden an die Gruppe CHOH gebundenen Reste, die identisch oder voneinander verschieden sind, und im Falle der tertiären Alkohole, die drei an die Gruppe COH gebundenen Gruppen, die identisch oder voneinander verschieden sind, Gruppen darstellen, die unter den geradkettigen oder verzweigten Alkylgruppen mit bis zu 4 Kohlenstoffatomen und Phenyl ausgewählt sind;
und
(3i) Lösungen von Alkoholaten (i) oder (2i) in den Monoalkoholen, aus denen sie gebildet sind;
und dadurch, daß ggf. Gemische auf der Basis von Aminovinylketonen gebildet werden, die neben der Verbindung VI insbesondere ein Pyrimidin der allgemeinen Formel 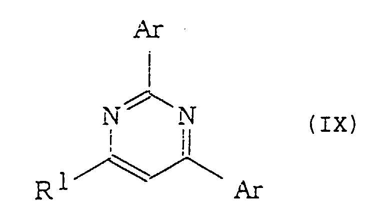 enthalten, worin die Gruppen Ar, die identisch sind, die folgende Gruppe bedeuten: 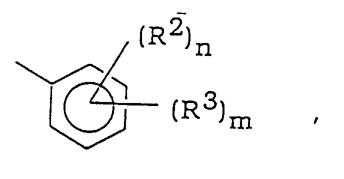 und die Gruppe R¹ die oben für die Aminovinylketone der Formel VI nach einem der Ansprüche 1 bis 5 angegebenen allgemeinen oder bevorzugten Bedeutungen aufweist,
- wobei für die Verbindung IX folgende Maßgaben gelten:
• (a) wenn n 0 und m 0 ist oder (b) wenn n 0 und m 1 oder 2 ist und R³ eine Alkylgruppe, Alkoxygruppe oder ein Halogenatom bedeutet, ist in beiden Fällen (a) oder (b) R¹ von einer Alkylgruppe oder einer Gruppe der Formel (R⁵-)ₚ-Phenyl-(-CR⁶R⁷)_{q}-, worin q 0 ist, verschieden;
• (c) wenn n 1 und m 0 ist und R² NO₂ bedeutet, ist R¹ von einer Gruppe (R⁵-)ₚ-Phenyl-(-CR⁶R⁷)_{q}-, worin q 0 ist, verschieden.

7. Verfahren nach Anspruch 6, dadurch gekennzeichnet, daß das Lösungsmittelmedium aus einem inerten Lösungsmittel oder einem Gemisch von inerten Lösungsmitteln besteht, die unter den polaren, aprotischen und nicht mit Wasser mischbaren Lösungsmitteln ausgewählt sind.

8. Verfahren nach einem der Ansprüche 6 und 7, dadurch gekennzeichnet, daß die Umsetzung mit den folgenden molaren Mengenanteilen der Bestandteile durchgeführt wird:
- aromatisches Nitril der Formel VIII : 1 mol
- R¹-Methylketon der Formel VII : 1 bis 4 mol
- Alkoholat : 1 bis 4 mol.

9. Verfahren nach einem der Ansprüche 6 bis 8, dadurch gekennzeichnet, daß die Kondensation in Gegenwart einer starken Base durchgeführt wird, die ausgewählt ist unter:
(4i) den Alkoholaten, die von Alkalimetallen und tertiären Monoalkoholen abgeleitet sind, wobei in der Struktur der Monoalkohole jeder der drei an die Gruppe COH gebundenen Reste, die identisch oder voneinander verschieden sind, unter einer geradkettigen Alkylgruppe mit 1 oder 2 Kohlenstoffatomen und Phenyl ausgewählt ist;
und
(5i) den Lösungen der Alkoholate (4i) in den Monoalkoholen, aus denen sie gebildet sind.

10. Verfahren nach Anspruch 9, dadurch gekennzeichnet, daß die Alkoholate Natriumalkoholate und Kaliumalkoholate sind, die von den folgenden Alkoholen abgeleitet sind: (CH₃)₃-COH, (CH₃)₂(C₆H₅) -COH und (C₆H₅)₃-COH.

11. Verfahren nach einem der Ansprüche 6 bis 10, dadurch gekennzeichnet, daß die Umsetzung bei einer Temperatur im Bereich von 30 bis 120 °C durchgeführt wird.

12. Verwendung der β-Aminovinylketone der Formel VI nach einem der Ansprüche 1 bis 5 oder Gemischen auf der Basis von Aminovinylketonen zur Herstellung von β-Diketonen der allgemeinen Formel 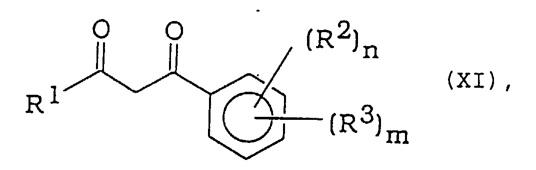 worin R¹, R², R³, n und m eine der für die Formel VI in einem der Ansprüche 1 bis 5 angegebenen allgemeinen oder bevorzugten Bedeutungen aufweisen, wobei die Verwendung dadurch gekennzeichnet ist, daß sie den Arbeitsschritt umfaßt, ein β-Aminovinylketon der Formel VI oder ein Gemisch auf Aminovinylketonbasis einer Hydrolyse zu unterziehen, indem ggf. in einem inerten Lösungsmittel in Gegenwart von Wasser und einer starken anorganischen oder organischen Säure gearbeitet wird.

13. Verwendung nach Anspruch 12, dadurch gekennzeichnet, daß in einem Lösungsmittelmedium gearbeitet wird, das mit dem Medium identisch ist, das im Rahmen des Verfahrens zur Herstellung der β-Aminovinylketone der Formel VI nach Anspruch 6 verwendet wird.

14. Verwendung nach Anspruch 13, dadurch gekennzeichnet, daß das Lösungsmittelmedium aus einem inerten Lösungsmittel oder einem Gemisch von inerten Lösungsmitteln besteht, wobei die Lösungsmittel unter den polaren, aprotischen und nicht mit Wasser mischbaren Lösungsmitteln ausgewählt sind.

15. Verwendung nach einem der Ansprüche 12 bis 14, dadurch gekennzeichnet, daß die Umsetzung mit den folgenden Mengenverhältnissen der Bestandteile durchgeführt wird:
- β-Aminovinylketon der Formel VI : 1 mol
- Wasser 1 bis 20 mol
- starke Säure 1 bis 6 Ionen H⁺.

16. Verwendung nach einem der Ansprüche 12 bis 15, dadurch gekennzeichnet, daß die starke Säure eine Säurekonstante in Wasser, pKs, von höchstens 3 aufweist.

17. Verwendung nach Anspruch 16, dadurch gekennzeichnet, daß die Säure unter den anorganischen Säuren, Organosulfonsäuren, Organophosphonsäuren und halogenierten Carbonsäuren ausgewählt ist.

18. Verwendung nach einem der Ansprüche 12 bis 17, dadurch gekennzeichnet, daß die Hydrolyse bei einer Temperatur im Bereich von 40 bis 120 °C durchgeführt wird.

## Claims

1. β-Aminovinyl ketones of general formula: 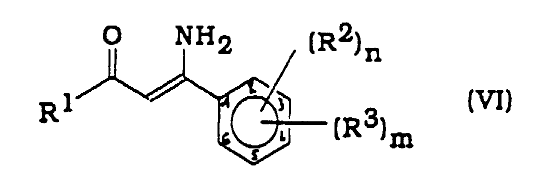 in which:
- R¹ represents:
* a straight- or branched-chain alkenyl or alkynyl group containing up to 6 carbon atoms which is optionally substituted with one or a number of halogen atom(s) and whose unsaturation is not conjugated with the * ketone double bond;
* a cycloalkyl group containing 3 to 6 carbon atoms, optionally substituted with one or a number of R⁴ or OR⁴ group(s) or one or a number of halogen atom(s);
* a cycloalkenyl group containing 4 to 6 carbon atoms, optionally substituted with one or a number of R⁴ or OR⁴ group(s) or one or a number of halogen atom(s), in which the ethylenic unsaturation is not conjugated with the ketone double bond;
* a group of formula (R⁵)ₚ-phenyl-(CR⁶R⁷)_{q}-;
* R⁴ being a straight- or branched-chain alkyl group containing up to 4 carbon atoms;
* R⁵ being a straight- or branched-chain alkyl group containing up to 4 carbon atoms or a halogen atom (F, Cl, Br or I);
* R⁶ and R⁷, which can be identical or different, each representing a straight- or branched-chain alkyl group containing up to 4 carbon atoms;
* p and q being integers, which can be identical or different, ranging from 0 to 3;
- R² represents an electron-withdrawing substituent chosen from the group formed by:
* a nitro group;
* a COR⁴ acyl group;
* a COOR⁸ group;
* an SR⁴ alkylthio group;
* a CONR⁸R⁹ carbamoyl or CSNR⁸R⁹ thiocarbamoyl group;
* a linear or branched haloalkyl or haloalkoxy group having up to 4 carbon atoms;
* R⁴ having the general definitions shown above;
* R⁸ and R⁹, which can be identical or different, each representing a hydrogen atom or a straight- or branched-chain alkyl group containing up to 4 carbon atoms;
- R³ represents:
* straight- or branched-chain alkyl or alkoxy group containing up to 4 carbon atoms, it being possible for the alkyl group to be optionally substituted with an OR⁴ group;
* a halogen atom;
* a group of formula O-(-CH₂)ᵣ-OR⁴;
* R⁴ possessing the general definition shown above and r being an integer ranging from 1 to 4;
- the n symbol represents an integer ranging from 0 to 2 and the m symbol represents an integer ranging from 0 to 3;
- with the following provisos a) and b):
a) R⁵ is other than a chlorine atom when R³ is a chlorine atom and q = 0,
b) when n, m and q are all equal to zero, then p is other than zero.

2. β-Aminovinyl ketones according to claim 1, characterized in that they correspond to the formula (VI) in which the n symbol represents a number ranging from 1 to 2 and the m symbol represents a number ranging from 0 to 3.

3. β-Aminovinyl ketones according to claim 2, characterized in that these are compounds in the structure of which:
- R¹ represents a cycloalkyl group containing 3 to 6 carbon atoms, optionally substituted with one or a number of R⁴ or OR⁴ group(s);
- R² represents an SR⁴ alkylthio group or a linear or branched haloalkyl or haloalkoxy group having up to 4 carbon atoms;
- R³ represents a straight- or branched-chain alkyl or alkoxy group containing up to 4 carbon atoms or a halogen atom.

4. β-Aminovinyl ketones according to claim 3, characterized in that these are compounds in the structure of which :
- the n symbol represents an integer equal to 1 or 2 and the m symbol represents an integer ranging from 0 to 2, with the additional condition according to which the sum of m+n is not more than 3;
- the group or one of the groups represented by (R²)ₙ is in the 2-position on the phenyl ring whereas the optional other group(s) represented by (R²)ₙ and/or (R³)ₘ are in the 3- and/or 4-position on the phenyl ring.

5. β-Aminovinyl ketones according to claim 4, characterized in that these are compounds in the structure of which :
- R¹ represents a cyclopropyl or 1-methylcyclopropyl group;
- R² represents a methylthio or ethylthio group or a trifluoromethyl or trifluoromethoxy group;
- R³ represents a methyl, ethyl, methoxy or ethoxy group or a chlorine, bromine or fluorine atom.

6. Process for the preparation of the β-aminovinyl ketones of formula (VI) according to any one of claims 1 to 5, characterized in that it comprises the condensation reaction of a methyl organo ketone of formula: 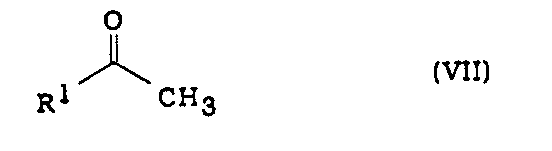 in which R¹ has one of the general or preferred definitions shown above with respect to the formula (VI), with an aromatic nitrile of formula: 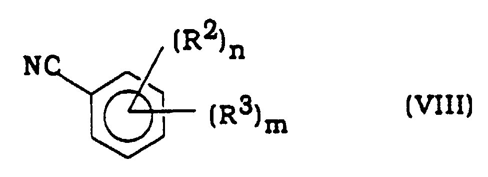 in which the R², R³, n and m symbols have one of the general or preferred definitions shown above with respect to the formula (VI), the said condensation reaction being carried out under an inert atmosphere, with prolonged heating, in inert solvent medium, in the presence of a strong base taken from the group formed by:
(i) alkoxides derived from alkali metals and primary monoalcohols in the structure of which the residue associated with the CH₂OH group is a branched-chain group in which the carbon atom directly bonded to the CH₂OH group is itself bonded to 2 or 3 other carbon atoms, these other carbon atoms belonging to identical or different radicals each chosen from a linear or branched alkyl radical having up to 3 carbon atoms and a phenyl radical;
(2i) alkoxides derived from alkali metals and secondary or tertiary monoalcohols in the structure of which each of the 2 identical or different residues associated with the CHOH group, in the case of secondary alcohols, or each of the 3 identical or different residues associated with the COH group, in the case of tertiary alcohols, consists of a radical chosen from a linear or branched alkyl radical having up to 4 carbon atoms and a phenyl radical; and
(3i) solutions of the (i) or (2i) alkoxides in the monoalcohols in which they have been prepared, and in that it optionally produces mixtures based on aminovinyl ketones comprising, in addition to the compound VI, in particular a pyrimidine of general formula: 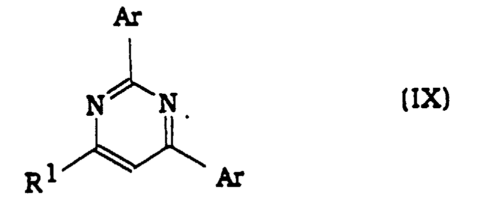
- in which the Ar symbols, which are identical to each other and represent the group: 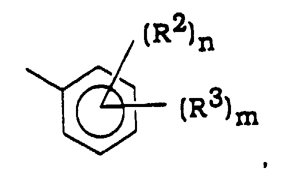 and the R¹ symbol have the general or preferred definitions shown above with respect to the aminovinyl ketone of formula (VI) in any one of claims 1 to 5,
- with the following provisos for this compound IX:
* (a) when n = 0 and m = 0 or (b) when n = 0 and m = 1 or 2 and R³ represents an alkyl or alkoxy group or a halogen atom, R¹ in each case (a) or (b) is then other than an alkyl group or than a group of formula (R⁵-)ₚ- phenyl-(-CR⁶R⁷)_{q}- where q = 0;
* (c) when n = 1 and m = 0 and R² represents NO₂, R¹ is then other than a group of formula (R⁵-)ₚ-phenyl(-CR⁶R⁷)_{q}- where q = 0.

7. Process according to claim 6, characterized in that the solvent medium consists of an inert solvent or a mixture of inert solvents chosen from water-immiscible aprotic polar solvents.

8. Process according to either of claims 6 and 7, characterized in that the reaction is carried out with the following molar proportions of ingredients:
- aromatic nitrile of formula (VIII) : 1 mol
- R¹ methyl ketone of formula (VII) : 1 to 4 mol
- alkoxide : 1 to 4 mol.

9. Process according to any one of claims 6 to 8, characterized in that the condensation reaction is carried out in the presence of a strong base taken from the group formed by :
(4i) alkoxides derived from alkali metals and tertiary monoalcohols in the structure of which each of the 3 identical or different residues associated with the COH group is a radical chosen from a linear alkyl radical having 1 or 2 carbon atoms and a phenyl radical; and
(5i) solutions of the (4i) alkoxides in the monoalcohols in which they have been prepared.

10. Process according to claim 9, characterized in that the targeted alkoxides are the sodium and potassium alkoxides derived from the following alcohols of formula: (CH₃)₃-COH, (CH₃)₂(C₆H₅) -COH and (C₆H₅)₃-COH.

11. Process according to any one of claims 6 to 10, characterized in that the reaction is carried out at a temperature lying in the range from 30°C to 120°C.

12. Use of the β-aminovinyl ketones of formula (VI) according to any one of claims 1 to 5 or of mixtures based on aminovinyl ketones in the preparation of β-diketones of general formula: 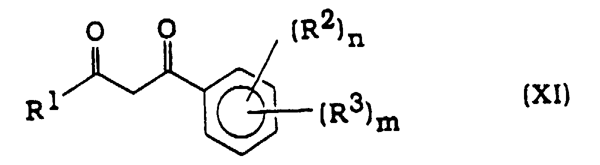 in which the R¹, R², R³, n and m symbols have one of the general or preferred definitions shown above, with respect to the formula (VI), in any one of claims 1 to 5, the said use being characterized in that it comprises subjecting a β-aminovinyl ketone of formula (VI) or a mixture based on aminovinyl ketone to a hydrolysis reaction, the hydrolysis reaction being carried out, optionally in an inert solvent medium, in the presence of water and a strong inorganic or organic acid.

13. Use according to claim 12, characterized in that the hydrolysis reaction is carried out in a solvent medium which is identical to that used in the context of the process for the preparation, according to claim 6, of the β-aminovinyl ketones of formula (VI).

14. Use according to claim 13, characterized in that the solvent medium consists of an inert solvent or a mixture of inert solvents chosen from water-immiscible aprotic polar solvents.

15. Use according to any one of claims 12 to 14, characterized in that the reaction is carried out with the following proportions of ingredients:
- β-aminovinyl ketone of formula (VI) : 1 mol
- Water : 1 to 20 mol
- Strong acid : 1 to 6 H⁺ ions.

16. Use according to any one of claims 12 to 15, characterized in that the strong acid has an ionization constant in water, pKa, which is less than or equal to 3.

17. Use according to claim 16, characterized in that the acid is chosen from the group consisting of inorganic acids, organosulphonic acids, organophosphonic acids and halogenated carboxylic acids.

18. Use according to any one of claims 12 to 17, characterized in that the hydrolysis reaction is carried out at a temperature lying in the range from 40°C to 120°C.
